(19) Europäisches Patentamt / European Patent Office / Office européen des brevets

(11) **EP 4 094 792 A1**

(12) **EUROPEAN PATENT APPLICATION**
published in accordance with Art. 153(4) EPC

(43) Date of publication:
**30.11.2022 Bulletin 2022/48**

(21) Application number: **20914937.6**

(22) Date of filing: **04.03.2020**

(51) International Patent Classification (IPC):
***A61M 11/00*** (2006.01)

(52) Cooperative Patent Classification (CPC):
**A61M 11/00**

(86) International application number:
**PCT/CN2020/073391**

(87) International publication number:
**WO 2021/146885 (29.07.2021 Gazette 2021/30)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.01.2020 CN 202020126768 U**

(71) Applicant: **Feellife Health Inc.
Shenzhen, Guangdong 518000 (CN)**

(72) Inventor: **HUA, Jian
Shenzhen, Guangdong 518000 (CN)**

(74) Representative: **Purdylucey Intellectual Property
6-7 Harcourt Terrace
D02 FH73 Dublin 2 (IE)**

(54) **QUANTITATIVE DOSING SELF-ADAPTIVE ATOMIZATION SYSTEM WITH MEMORY FUNCTION**

(57)    Disclosed is a quantitative dosing self-adaptive atomization system with a memory function. The system comprises a liquid storage tank (1) and an atomization bin (4), wherein liquid in the liquid storage tank (1) flows into the atomization bin (4) and is atomized in the atomization bin (4). The system further comprises a peristaltic pump (2), wherein the peristaltic pump (2) pumps the liquid in the liquid storage tank (1) into the atomization bin (4). The atomization system can control the amount of liquid medicine pumped to the atomization bin (4) by means of the peristaltic pump (2).

FIG. 3

EP 4 094 792 A1

**Description**

**TECHNICAL FIELD**

The disclosure relates to the field of atomizers, and more particularly, to a self-adaptive atomization system for quantitative administration with a memory function.

**BACKGROUND**

Atomizer is a device for atomizing a fluid into fine particles to form mist. At present, the atomizer is used for atomizing water so as to be used as a humidifier, or the atomizer is used for atomizing a medicine to form medicine mist for a patient to take in. At present, there are three main atomization principles, corresponding to ultrasonic atomizer, compression atomizer, and mesh atomizer respectively. No matter what kind of atomizers they are, a liquid storage tank for storing liquid medicine to be atomized and an atomization wafer for atomizing the liquid in the liquid storage tank are needed. Under the control of an atomizer controller, the liquid in the liquid storage tank continuously flows into the atomization wafer for atomization. Such atomizer can only control a total volume of the liquid storage tank, but cannot control a dosage of the atomized liquid separately. For some patients who take the atomized medicine, the atomizer cannot control a dosage of the medicine well, thus being unable to meet requirements of users.

**SUMMARY**

Aiming at the deficiencies that a current atomizer cannot control a dosage of atomized liquid at any time and cannot meet needs of a user, the disclosure provides a self-adaptive atomization system for quantitative administration with a memory function, which uses a peristaltic pump to control an atomization amount.

The technical solutions used in the disclosure to achieve the technical objects are as follows: a self-adaptive atomization system for quantitative administration with a memory function includes a liquid storage tank and an atomization bin, where liquid in the liquid storage tank flows into the atomization bin and is atomized in the atomization bin; and the system further includes a peristaltic pump configured for pumping the liquid in the liquid storage tank into the atomization bin.

In the disclosure, the peristaltic pump may be used to control an amount of liquid medicine pumped into the atomization bin, thus overcoming the deficiencies of the current atomizer and meeting the needs of the user.

Further, the self-adaptive atomization system for quantitative administration with a memory function above further includes a measuring system for measuring an atomization rate, where the measuring system includes a measuring device for measuring an outflow speed of the liquid in the liquid storage tank and a main control IC for processing measured data of the measuring device to finally obtain the atomization rate, and measurement output ends of the measuring device are respectively sampled by a sampling circuit and then connected to corresponding pins of the main control IC.

Further, in the self-adaptive atomization system for quantitative administration with a memory function above, the measuring device for measuring an outflow speed of the liquid in the liquid storage tank is a flow sensor U8 mounted at a front end of an atomization wafer in the atomization bin, and the sampling circuit includes an operational amplifier U9; and an output signal of the flow sensor U8 is amplified by the operational amplifier U9 and then connected to the corresponding pins of the main control IC.

Further, in the self-adaptive atomization system for quantitative administration with a memory function above, the measuring device for measuring an outflow speed of the liquid in the liquid storage tank is a pressure sensor U6 which has a sensing surface arranged at a bottom of the liquid storage tank and is contacted with the liquid, and data output by the pressure sensor U6 and indicating a pressure at the bottom of the liquid storage tank are directly connected to the corresponding pins of the main control IC.

Further, the self-adaptive atomization system for quantitative administration with a memory function above further includes a display screen controlled by the main control IC.

The disclosure is further described hereinafter with reference to the drawings and the specific implementations.

**BRIEF DESCRIPTION OF THE DRAWINGS**

FIG. 1 is a stereoscopic diagram of a self-adaptive atomization system for quantitative administration with a memory function according to the disclosure.

FIG. 2 is a front view of the self-adaptive atomization system for quantitative administration with a memory function according to the disclosure.

FIG. 3 is a cross-sectional view of A-A in FIG. 2.

FIG. 4 is a control block diagram of the self-adaptive atomization system for quantitative administration with a memory function according to the disclosure.

FIG. 5 is a sampling circuit diagram of a flow sensor.

FIG. 6 is a sampling circuit diagram of a pressure sensor.

DETAILED DESCRIPTION

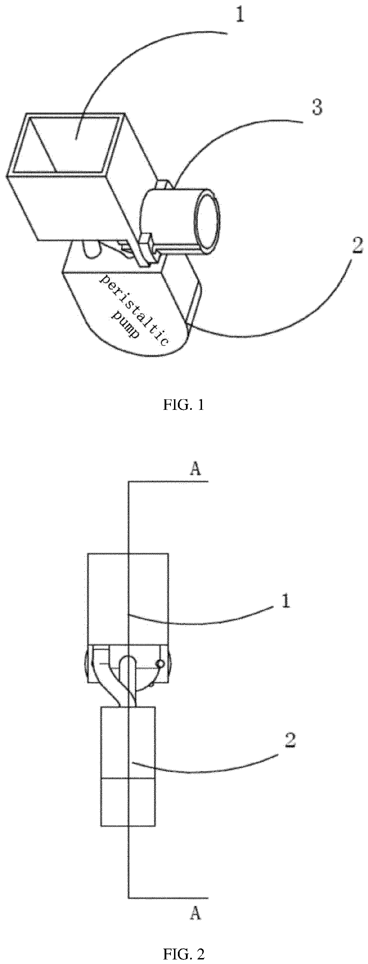

Embodiment 1 is a self-adaptive atomization system for quantitative administration with a memory function, which can effectively control an amount of atomized liquid, and as shown in FIG. 1, FIG. 2 and FIG. 3, the whole self-adaptive atomization system for quantitative administration with a memory function includes a liquid storage tank 1, a peristaltic pump 2 and an atomization bin 3. A liquid medicine in the liquid storage tank 1 is pumped into an atomization bin 4 of the atomization bin 3 by the peristaltic pump 2, and atomized in the atomization bin 4.

In the embodiment, the liquid medicine is loaded into the liquid storage tank 1 first, the liquid medicine flows through the peristaltic pump 2, the peristaltic pump 2 controls the addition of the liquid medicine, and an amount of the added liquid is controlled by a signal sent to the peristaltic pump 2 by a mechanical switch. A user may adjust the amount of the added liquid according to a scale, such as 100 mg to 1,000 mg, and then the added liquid directly enters the atomization bin 4 for atomization.

In the embodiment, a flow sensor or a pressure sensor is used to detect parameters during atomization, and an atomization rate and a predicted atomization amount in a period of time are obtained through a series of calculations. According to the predicted atomization amount, the peristaltic pump is used to accurately supply the liquid, and a total amount of currently supplied liquid and a volume of unfinished liquid are displayed on a display screen. A given maximum atomization amount is set for a specific medicine.

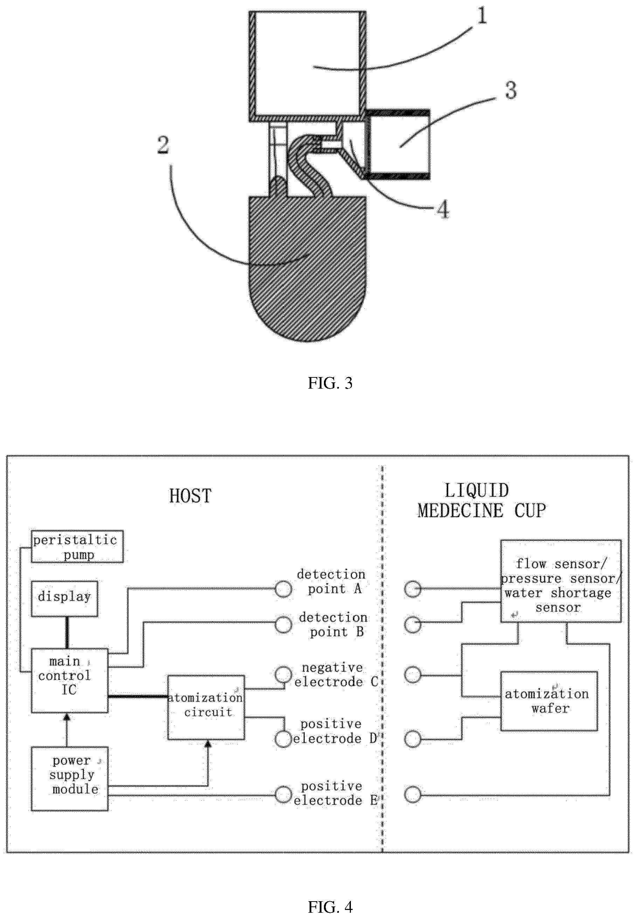

A detection control circuit of the self-adaptive atomization system for quantitative administration with a memory function in the embodiment is shown in FIG. 4. The parameters during atomization detected by the flow sensor or the pressure sensor are processed through a main control IC, and an atomization rate of the liquid medicine in the self-adaptive atomization system for quantitative administration with a memory function may be output, thus being convenient for a patient to effectively control a medicine dosage. Relevant data may be displayed on the display screen. In practice, the power supplied to the self-adaptive atomization system for quantitative administration with a memory function may also be effectively controlled through the main control IC, and the main control IC sets the power supplied to the self-adaptive atomization system for quantitative administration with a memory function according to the parameters during atomization detected by the flow sensor or the pressure sensor.

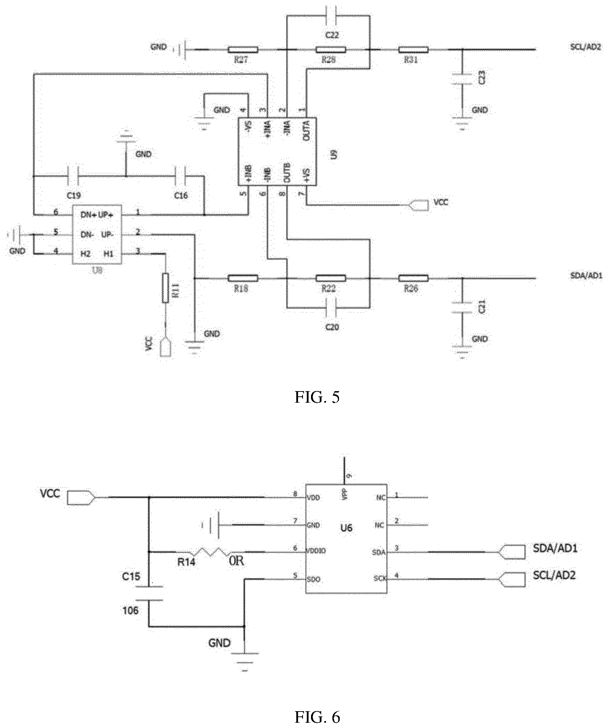

FIG. 5 shows a detection circuit where the parameters during atomization are detected by the flow sensor, and the flow sensor U8 is placed at a front end of an atomization wafer. When the liquid flows over a surface of the flow sensor U8, an output signal may be changed. The faster the flow is, the greater the change of the signal will be. As shown in FIG. 5, pin 5 and pin 4 of the flow sensor U8 are grounded, pin 3 is connected to a working power supply through a current limiting resistor R11, and pin 1, pin 2 and pin 6 are used for outputting signals, where pin 1 and pin 2 output balanced signals, and pin 6 outputs a balanced signal to the ground. A chip U9 includes two operational amplifiers, where an operational amplifier A amplifies the signal output by pin 6, and an operational amplifier B amplifies the balanced signals output by pin 1 and pin 2.

The pin 6 (DN+) of the flow sensor U8 is connected to a non-inverting input terminal +INA of the operational amplifier A in the chip U9, which is namely pin 3. An inverting input terminal INA of the operational amplifier A, which is namely pin 2, is connected to an output terminal OUTA (pin 1) through a resistor R28 and a capacitor C22. Meanwhile, the inverting input terminal INA of the operational amplifier A is also grounded through a resistor R27, and the output terminal OUTA of the operational amplifier A outputs a signal SCL/AD2 to a corresponding pin of the main control IC via a current limiting resistor R31.

The same also applies for the amplification of the balanced signals output by pin 1 and pin 2, except that an inverting input terminal INB of the operational amplifier B is connected to pin 2 ( UP-) of the flow sensor U8 through a resistor R18, and other connections are basically the same. That is to say, pin 1 (UP+) of the flow sensor U8 is connected to a non-inverting input terminal +INB of the operational amplifier B, and the inverting input terminal INB of the operational amplifier B, which is namely pin 6 of the U8, is connected to an output terminal OUTB (pin 8) through a resistor R22 and a capacitor C20. The output terminal OUTB of the operational amplifier B outputs a signal SDA/AD1 to a corresponding pin of the main control IC through a current limiting resistor R26. The flow sensor U8 includes one resistance heater and two thermocouples (thermopiles), and each thermopile is symmetrically located on upstream and downstream positions of the heater. The upstream thermopile is cooled by a flowing medium, and the downstream thermopile is heated by heat transfer from the heater to a flowing direction. The output signal is a differential voltage between the upstream and downstream thermopiles. Specifically, pins H1 and H2 of the chip refer to the heater; DN+ refers to the downstream thermopile+; DN- refers to the downstream thermopile-; UP+ refers to the upstream thermopile+; and UP- refers to the upstream thermopile-.

In addition, the pin 6 (DN+) and pin 1 (UP+) of the flow sensor U8 are grounded through a capacitor C19 and a capacitor C16 respectively, which introduce an alternate current into the ground. The output signal SCL/AD2 and the output signal SDA/AD1 are grounded through the capacitor C22 and the capacitor C21 respectively, which realizes an alternate current being grounded.

The signals are amplified by the operational amplifiers and then output to two points SCL/AD1 and SDA/AD2, and the two points are connected to a host through contacts A and B.

The main control IC may judge a flow velocity of the liquid by detecting signals of the two points. Meanwhile, data may be statistically analyzed to obtain the atomization amount per unit time.

Setting a flow velocity V(t) of the liquid corresponding to data AD1 collected at a certain moment t by a circuit, then a flow rate Q in a period of time [T1, T2] is a definite integral of the V(t) in the interval [T1, T2]:

$$Q = \text{QUOTE} \int_{T1}^{T2} V(t)dt \int_{T1}^{T2} V(t)dt$$

The main control IC constantly and quickly detects the flow velocity of the liquid, and it is considered that the flow in a short period of time $\Delta t$ is constant, and the flow in the short period of time is that $Q1=V1*\Delta t$. The main control accumulates the flow of each time slice to achieve an effect of approaching the integral, so as to obtain the flow in the period of time [T1, T2], and derive an average velocity that is $v=Q/(T2-T1)$. Due to structural restriction, all liquids passing through the atomization wafer should pass through the flow sensor first, so that the atomization rate is equal to the measured flow velocity v of the liquid.

In addition, a mist spray speed may also be calculated by measuring a pressure at a bottom of the liquid storage tank 1. As shown in FIG. 6, U6 is a pressure sensor, and a sensing surface is placed at the bottom of the liquid storage tank to contact with the liquid. When a liquid level of the liquid storage tank rises or falls, a pressure on the pressure sensor may be changed. Output of the pressure sensor U6 may be directly connected to a corresponding pin of the main control IC without amplification.

As shown in FIG. 6, pin 8 (VDD) and pin 6 (VDDIO) of the pressure sensor U6 are connected to a power supply, and a current limiting resistor R14 is connected in front of pin 6 (VDDIO) only. The power supply VDD is further grounded through a capacitor C15, and the pressure sensor U6 is grounded through pin 7 and pin 5 respectively. As shown in FIG. 6, the pressure sensor U6 has a pin SDO for data output, a pin VDDIO for power supply input, a pin VPP for power supply grounding, a pin SDA for data of IIC, a pin SCK for a clock of IIC, and a suspended pin for a SPI interface.

In the embodiment, there is a correlation coefficient k between different pressures and corresponding liquid levels. The coefficient K may be calibrated by an actual test.

Meanwhile, for a specific product, a shape of the liquid storage tank 1 is fixed, and a corresponding volume may be derived by measuring the liquid level.

Accordingly, considering that the liquid storage tank 1 may have different inclination angles when used by a user, in order to measure a volume of the liquid medicine more accurately, the system is added with an angle sensor to measure the inclination angle of the liquid storage tank 1. In the case of knowing the inclination angle and the liquid level, the volume of the liquid may be accurately derived according to different shapes of the liquid storage tank.

Assuming that the volume of the liquid is reduced by $\Delta Q$ in a period of time $\Delta t$, then the atomization rate is that $v=\Delta Q/\Delta t$.

The main control IC reads the data collected by the pressure sensor through two contacts SD/AD1 and SD/AD2, thus deriving a real-time liquid level and a real time volume of the liquid medicine. The pressure is read at two time points T1 and T2 respectively, so as to calculate a volume difference $\Delta Q$, thus further obtaining an average atomization rate $\Delta Q/(T2-T1)$ from T1 to T2.

Accordingly, after the atomization rate is measured by either of the above two methods, the atomization rate may be accurately controlled through a solution of increasing and reducing the atomization rate by adjusting a frequency and a duty ratio and other methods described above. For example, the atomization rate is preset as 0.6 ml/min. If the actually measured atomization rate is excessively large, the preset atomization rate is reduced by adjusting the frequency or the duty ratio and other methods; and if the actually measured atomization rate is excessively is small, the preset atomization rate is increased. The adjustment is repeated, so as to realize a dynamic balance of the atomization rate.

The self-adaptive atomization system for quantitative administration with a memory function in the embodiment has the following characteristics:

1. a miniature precision peristaltic pump is provided internally for automatic adjustment of administration;

2. an electromagnetic valve is provided for automatic control of an administrating device;

3. there is a function of independently cleaning a pipeline;

4. an atomization gear may be arranged, the gear may be memorized, and the user may be self-adaptive to an atomization habit according to the memory function;

5. the user may preset the administration and may adjust the administration dosage at any time during use;

6. an independent liquid storage tank is provided, and the liquid storage tank is communicated with the atomization bin through puncturing; and

7. the opening is sealed with an aluminum foil.

**Claims**

1. A self-adaptive atomization system for quantitative administration with a memory function, comprising a liquid storage tank (1) and an atomization bin (4), wherein liquid in the liquid storage tank (1) flows into the atomization bin (4) and is atomized in the atomization bin (4); **characterized in that**, the system further comprises a peristaltic pump (2) configured for pumping the liquid in the liquid storage tank (1) into the atomization bin (4).

2. The self-adaptive atomization system for quantitative administration with a memory function according to claim 1, further comprising a measuring system for measuring an atomization rate, wherein the measuring system comprises a measuring device for measuring an outflow speed of the liquid in the liquid storage tank (1) and a main control IC for processing measured data of the measuring device to finally obtain the atomization rate, and measurement output ends of the measuring device are respectively sampled by a sampling circuit and then connected to corresponding pins of the main control IC.

3. The self-adaptive atomization system for quantitative administration with a memory function according to claim 2, wherein the measuring device for measuring an outflow speed of the liquid in the liquid storage tank (1) is a flow sensor U8 mounted at a front end of an atomization wafer in an atomization core (3), and the sampling circuit comprises an operational amplifier U9; and an output signal of the flow sensor U8 is amplified by the operational amplifier U9 and then connected to the corresponding pins of the main control IC.

4. The self-adaptive atomization system for quantitative administration with a memory function according to claim 2, wherein the measuring device for measuring an outflow speed of the liquid in the liquid storage tank (1) is a pressure sensor U6 which has a sensing surface arranged at a bottom of the liquid storage tank (1) and is contacted with the liquid, and data output by the pressure sensor U6 and indicating a pressure at the bottom of the liquid storage tank (1) are directly connected to the corresponding pins of the main control IC.

5. The self-adaptive atomization system for quantitative administration with a memory function according to claim 2, 3 or 4, further comprising a display screen controlled by the main control IC.

1

3

2

peristaltic
pump

FIG. 1

A

1

2

A

FIG. 2

FIG. 3

FIG. 4

FIG. 5

FIG. 6

## INTERNATIONAL SEARCH REPORT

| International application No. |
|---|
| **PCT/CN2020/073391** |

| A. | CLASSIFICATION OF SUBJECT MATTER |
|---|---|

A61M 11/00(2006.01)i

According to International Patent Classification (IPC) or to both national classification and IPC

| B. | FIELDS SEARCHED |
|---|---|

Minimum documentation searched (classification system followed by classification symbols)

A61M11/-, A61M15/-

Documentation searched other than minimum documentation to the extent that such documents are included in the fields searched

Electronic data base consulted during the international search (name of data base and, where practicable, search terms used)

WPI, EPODOC, CNKI, CNPAT: 雾化, 给药, 用药, 加药, 定量, 蠕动泵, 流速, 压力, 湿度, 传感, 检测, 电路; atomiz+, medicat +, dosage, quantitative, peristaltic pump, velocity, pressure, humid+, moist+, sensor, detect+, circuit

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 204337468 U (THE FOURTH MILITARY MEDICAL UNIVERSITY OF PLA) 20 May 2015 (2015-05-20)<br>description, paragraphs [0004]-[0033], and figures 1-4 | 1-5 |
| A | CN 209864908 U (SUZHOU KOWLOON HOSPITAL CO., LTD.) 31 December 2019 (2019-12-31)<br>entire document | 1-5 |
| A | CN 110613604 A (THE FIRST AFFILIATED HOSPITAL OF SHANTOU UNIVERSITY MEDICAL COLLEGE) 27 December 2019 (2019-12-27)<br>entire document | 1-5 |
| A | CN 206063480 U (ZHENGZHOU HEJI BIOLOGICAL TECHNOLOGY CO., LTD.) 05 April 2017 (2017-04-05)<br>entire document | 1-5 |
| A | GB 2488992 A (ADVANCED OPHTHALMIC PHARMA LTD.) 19 September 2012 (2012-09-19)<br>entire document | 1-5 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 September 2020** | **27 September 2020** |

| Name and mailing address of the ISA/CN | Authorized officer |
|---|---|
| **China National Intellectual Property Administration (ISA/ CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2020/073391**

| Patent document cited in search report | | | Publication date (day/month/year) | Patent family member(s) | Publication date (day/month/year) |
|---|---|---|---|---|---|
| CN | 204337468 | U | 20 May 2015 | None | |
| CN | 209864908 | U | 31 December 2019 | None | |
| CN | 110613604 | A | 27 December 2019 | None | |
| CN | 206063480 | U | 05 April 2017 | None | |
| GB | 2488992 | A | 19 September 2012 | WO  2012101634  A2 | 02 August 2012 |

Form PCT/ISA/210 (patent family annex) (January 2015)